# EUROPEAN PATENT APPLICATION

(11) **EP 2 829 234 A2**
(43) Date of publication of application: **28.01.2015**
(21) Application number: 14178701.0
(22) Date of filing: 28.07.2014
(51) Int. Cl.: A61B 8/00

(54) **Ultrasound apparatus and method of generating ultrasound image**

(30) Priority: 26.07.2013 KR 20130088981
(71) Applicant: Samsung Electronics Co., Ltd, Gyeonggi-do 443-742 (KR)
(72) Inventor: Cho, Jeong, Seoul (KR); Han, Ho-san, Seoul (KR)
(74) Representative: Land, Addick Adrianus Gosling

(57) **Abstract**

A method of generating an ultrasound image in an ultrasound apparatus connected with at least one wired probe and at least one wireless probe is provided. A wired response signal corresponding to a first ultrasound response signal reflected from an object is received from the at least one wired probe, and a wireless response signal corresponding to a second ultrasound response signal reflected from the object is received from the at least one wireless probe. An ultrasound image of the object is generated by using the wired and wireless response signals.

## Description

This application claims priority from Korean Patent Application No. 10-2013-0088981, filed on July 26, 2013, in the Korean Intellectual Property Office, the disclosure of which is incorporated herein in its entirety by reference.

Methods and apparatuses consistent with the exemplary embodiments relate to medical imaging, and more particularly, to an ultrasound apparatus and a method of generating an ultrasound image of an object.

Ultrasound apparatuses are equipment for observing the internal structure of an organic body. Ultrasound apparatuses are non-invasive test devices, and show structural details, tissues, and the flow of a fluid inside a body.

An ultrasound apparatus irradiates an ultrasound signal generated by a transducer of a probe onto an object, receives an ultrasound response signal reflected from the object, and thus obtains an image of the inside of the object. In particular, an ultrasound apparatus is used for medical purposes, for example, observation, detection of a foreign substance, and diagnosis of an injury inside an object.

Since ultrasound apparatuses are stable in comparison to other apparatuses such as X-ray and computed tomography (CT) apparatuses, do not cause radiation exposure and thus are safe, and display an image in real time, the ultrasound apparatuses are broadly used together with other image diagnosis apparatuses.

One or more exemplary embodiments include an ultrasound apparatus and a method of generating an ultrasound image of an object with an ultrasound apparatus including a wired probe and a wireless probe.

One or more exemplary embodiments include an ultrasound apparatus and a method of generating an ultrasound image of an object with an ultrasound apparatus including a wireless probe wirelessly connected to an external device.

Additional aspects will be set forth in part in the description which follows and, in part, will be apparent from the description, or may be learned by practice of the exemplary embodiments.

According to one or more exemplary embodiments, a method of generating an ultrasound image in an ultrasound apparatus connected with at least one wired probe and at least one wireless probe includes: receiving, from the at least one wired probe, a wired response signal corresponding to a first ultrasound response signal reflected from an object; receiving, from the at least one wireless probe, a wireless response signal corresponding to a second ultrasound response signal reflected from the object; and generating an ultrasound image of the object based on the wired and wireless response signals.

According to an aspect of the exemplary embodiment, the generating of the ultrasound image may include generating a wired ultrasound image of the object based on the wired response signal, and generating a wireless ultrasound image of the object based on the wireless response signal.

According to another aspect of the exemplary embodiment, the method may further include outputting the wired ultrasound image on a first display and outputting the wireless ultrasound image on a second display.

According to another aspect of the exemplary embodiment, the wireless ultrasound image may include an ultrasound image different from the wired ultrasound image.

According to another aspect of the exemplary embodiment, the generating of the ultrasound image may include generating a composite ultrasound image of the object by combining the wired ultrasound image and wireless ultrasound image.

According to another aspect of the exemplary embodiment, the generating of the ultrasound image may include generating wired ultrasound data corresponding to the wired response signal and wireless ultrasound data corresponding to the wireless response signal by processing the wired and wireless response signals in a time-sharing manner; and generating the wired ultrasound image based on the wired ultrasound data and generating the wireless ultrasound image based on the wireless ultrasound data.

According to another aspect of the exemplary embodiment, the generating of the ultrasound image may include generating wired ultrasound data from the wired response signal based on a first signal processor and generating wireless ultrasound data from the wireless response signal by using a second signal processor; and generating the wired ultrasound image based on the wired ultrasound data and generating the wireless ultrasound image based on the wireless ultrasound data.

According to aspect of the exemplary embodiment, the at least one wireless probe transmits the wireless response signal to an external device.

According to another aspect of the exemplary embodiment, the generating of the ultrasound image may include generating first wireless ultrasound data based on a first part of the wireless response signal received from the at least one wireless probe; receiving from the external device second wireless ultrasound data generated based on a second part of the wireless response signal transmitted to the external device; and generating a wireless ultrasound image of the object by combining the first wireless ultrasound data and the second wireless ultrasound data.

According to another aspect of the exemplary embodiment, the generating of the ultrasound image may include generating a first wireless ultrasound image based on the wireless response signal received from the at least one wireless probe; receiving from the external device a second wireless ultrasound image generated based on the wireless response signal transmitted to the external device; and generating a composite wireless ultrasound image of the object by combining the first wireless ultrasound image and the second wireless ultrasound image.

According to another aspect of the exemplary embodiment, the ultrasound apparatus may have a master control authority on the at least one wireless probe, and the external device may have a slave control authority on the at least one wireless probe.

According to another aspect of the exemplary embodiment, the at least one wireless probe is mapped to an external device, and the method may further include transmitting the wireless ultrasound image to the external device.

According to one or more exemplary embodiments, a method of generating an ultrasound image includes: receiving from at least one wireless probe a wireless response signal corresponding to an ultrasound response signal reflected from an object; generating first wireless ultrasound data based on a first part of the wireless response signal; receiving from an external device second wireless ultrasound data generated based on a second part of the wireless response signal; and generating a wireless ultrasound image of the object by combining the first wireless ultrasound data and the second wireless ultrasound data.

According to one or more exemplary embodiments, a method of generating an ultrasound image includes: receiving from at least one wireless probe a wireless response signal corresponding to an ultrasound response signal reflected from an object; generating a first wireless ultrasound image based on the wireless response signal; receiving from an external device a second wireless ultrasound image generated based on the wireless response signal; and generating a composite wireless ultrasound image of the object by combining the first wireless ultrasound image and the second wireless ultrasound image.

According to one or more exemplary embodiments, a method of generating an ultrasound image includes: receiving a wireless response signal of an object from at least one wireless probe mapped to an external device; generating a wireless ultrasound image of the object based on the wireless response signal; and transmitting the wireless ultrasound image to the external device, wherein the external device does not include an ultrasound apparatus.

According to one or more exemplary embodiments, a non-transitory computer-readable recording medium having recorded thereon a computer program for executing the method of generating the ultrasound image.

According to one or more exemplary embodiments, an ultrasound image generation apparatus comprising: at least one wired probe configured to obtain a wired response signal corresponding to a first ultrasound response signal reflected from an object; at least one wireless probe configured to obtain a wireless response signal corresponding to a second ultrasound response signal reflected from the object; and an image processor configured to generate an ultrasound image of the object based on the wired response signal and the wireless response signal received from the at least one wired probe and the at least one wireless probe.

According to another aspect of the exemplary embodiment, the image processor is configured to generate a wired ultrasound image of the object based on the wired response signal, and configured to generate a wireless ultrasound image of the object based on the wireless response signal.

According to another aspect of the exemplary embodiment, the ultrasound apparatus may further comprise a first display configured to output the wired ultrasound image; and a second display configured to output the wireless ultrasound image.

According to another aspect of the exemplary embodiment, the wireless ultrasound image may include an ultrasound image which is different from the wired ultrasound image.

According to another aspect of the exemplary embodiment, the image processor may generate a composite ultrasound image of the object by combining the wired ultrasound image and the wireless ultrasound image.

According to another aspect of the exemplary embodiment, the image processor comprises a signal processor configured to generate wired ultrasound data corresponding to the wired response signal and wireless ultrasound data corresponding to the wireless response signal by processing the wired response signal and the wireless response signal in a time-sharing manner; and an image generator configured to generate the wired ultrasound image based on the wired ultrasound data and generate the wireless ultrasound image based on the wireless ultrasound data.

According to another aspect of the exemplary embodiment, the image processor may include a first signal processor configured to generate wired ultrasound data by processing the wired response signal; a second signal processor configured to generate wireless ultrasound data by processing the wireless response signal; and an image generator configured to generate the wired ultrasound image based on the wired ultrasound data and generating the wireless ultrasound image based on the wireless ultrasound data.

According to another aspect of the exemplary embodiment, the at least one wireless probe may transmit the wireless response signal to an external device.

According to another aspect of the exemplary embodiment, the image processor may generate first wireless ultrasound data based on a first part of the wireless response signal received from the at least one wireless probe; receive, from the external device, second wireless ultrasound data generated based on a second part of the wireless response signal transmitted to the external device; and generate a wireless ultrasound image of the object by combining the first wireless ultrasound data and the second wireless ultrasound data.

According to aspect of the exemplary embodiment, the image processor may generate a first wireless ultrasound image based on the wireless response signal received from the at least one wireless probe; receive from the external device a second wireless ultrasound image generated based on the wireless response signal transmitted to the external device; and generate a composite wireless ultrasound image of the object by combining the first wireless ultrasound image and the second wireless ultrasound image.

According to another aspect of the exemplary embodiment, the ultrasound apparatus may have a master control authority on the at least one wireless probe, and the external device may have a slave control authority on the at least one wireless probe.

According to another aspect of the exemplary embodiment, the at least one wireless probe is mapped to an external device, and the ultrasound apparatus may further include a communication device configured to transmit the wireless ultrasound image to the external device.

### BRIEF DESCRIPTION OF THE DRAWINGS

These and/or other aspects will become apparent and more readily appreciated from the following description of the exemplary embodiments, taken in conjunction with the accompanying drawings of which:
FIGS. 1A and 1B are images of an ultrasound apparatus including a plurality of probes, according to an exemplary embodiment;
FIG. 2 is a flowchart of a method of generating an ultrasound image, according to an exemplary embodiment;
FIG. 3 is an image of an ultrasound apparatus including a wireless probe wirelessly connected to external devices, according to another exemplary embodiment;
FIG. 4 is a flowchart of a method of generating an ultrasound image, according to another exemplary embodiment;
FIG. 5 is a flowchart of a method of generating an ultrasound image, according to another exemplary embodiment;
FIG. 6 is a flowchart of a method of generating an ultrasound image, according to another exemplary embodiment;
FIG. 7A is a block diagram of an ultrasound apparatus according to an exemplary embodiment;
FIG. 7B is a block diagram of an ultrasound apparatus according to another exemplary embodiment;
FIG. 8A is a block diagram of an ultrasound apparatus according to another embodiment of the present invention;
FIG. 8B is a block diagram of an ultrasound apparatus according to another embodiment of the present invention; and
FIG. 9 is a block diagram of an ultrasound apparatus according to another exemplary embodiment.

### DETAILED DESCRIPTION OF EXEMPLARY EMBODIMENTS

The exemplary embodiments will now be described more fully with reference to the accompanying drawings, in which exemplary embodiments are shown. The exemplary embodiments may, however, be embodied in many different forms and should not be construed as being limited to the exemplary embodiments set forth herein; rather, these exemplary embodiments are provided so that this disclosure will be thorough and complete, and will fully convey the concept of the exemplary embodiments to one of ordinary skill in the art.

Terms used in the present specification are described briefly before explaining the exemplary embodiments.

All terms including descriptive or technical terms which are used herein should be construed as having meanings that are obvious to one of ordinary skill in the art. However, the terms may have different meanings according to an intention of one of ordinary skill in the art, precedent cases, or the appearance of new technologies. Also, some terms may be arbitrarily selected by the applicant, and in this case, the meaning of the selected terms will be described in detail in the detailed description of the exemplary embodiments. Thus, the terms used herein have to be defined based on the meaning of the terms together with the description throughout the specification.

Also, when a part "includes" or "comprises" an element, unless there is a particular description contrary thereto, the part can further include other elements, not excluding the other elements. In the following description, terms such as "unit" may be embodied as, but not limited to, software or a hardware component, such as a field programmable gate array (FPGA) or an application specific integrated circuit (ASIC). However, a unit may advantageously be configured to reside on an addressable storage medium and configured to execute one or more processors. Thus, a unit may include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. The functionality provided for in the components and units may be combined into fewer components and units or further separated into additional components and units.

In the specification, the expression "at least one of A and B" is used to cover a selection of A, a selection of B, or a selection of A and B. In another example, the expression "at least one of A, B, and C" is used to cover a selection of A, a selection of B, a selection of C, a selection of A and B, a selection of B and C, or a selection of A, B, and C. Even when more items are enumerated, it will be obvious to one of ordinary skill in the art that they can be definitely extendedly construed.

Throughout the specification, an "image" may mean multi-dimensional data including discrete image elements (e.g., pixels of a two-dimensional (2D) image and voxels of a three-dimensional (3D) image). For example, the image may include a medical image of an object which is obtained by using an X-ray diagnosis system, a computed tomography (CT) diagnosis system, a magnetic resonance imaging (MRI) diagnosis system, an ultrasound diagnosis system, or another medical diagnosis system.

Also, throughout the specification, an "object" may include a human, an animal, or a part of a human or animal. For example, the object may include organs such as liver, heart, womb, brain, breast, abdomen, or the like, or a blood vessel. Also, the object may include a phantom. The phantom means a material having a volume that is very close to a density and effective atomic number of an organism, and may include a sphere phantom having a characteristic similar to a physical body.

Throughout the specification, a "user" may be, but is not limited thereto, a medical expert including a doctor, a nurse, a medical laboratory technologist, a medial image expert, a radiologist, and a technician who repairs a medical apparatus.

The exemplary embodiments will now be described more fully with reference to the accompanying drawings, in which exemplary embodiments are shown. The exemplary embodiments may, however, be embodied in many different forms and should not be construed as being limited to the exemplary embodiments set forth herein; rather, these exemplary embodiments are provided so that this disclosure will be thorough and complete, and will fully convey the concept of the exemplary embodimentsto those of ordinary skill in the art. In the following description, well-known functions or constructions are not described in detail since they would obscure the exemplary embodiments with unnecessary detail.

In a general ultrasound system, a probe is connected to an ultrasound apparatus with a one-to-one manner and exchanges data with the ultrasound apparatus, and the ultrasound apparatus displays an ultrasound image that is generated by processing data received from the probe.

However, a probe of an ultrasound apparatus 100 according to the present embodiment may be simultaneously connected to the ultrasound apparatus 100 and an external device and may exchange data with them. Also, the ultrasound apparatus 100 according to the present embodiment may be simultaneously connected with a plurality of probes and may exchange data with them.

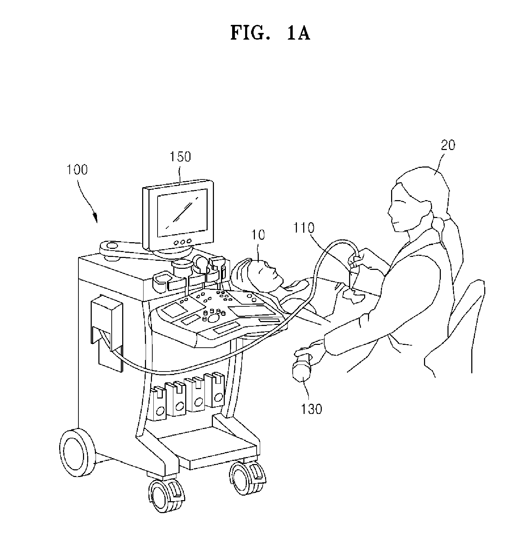

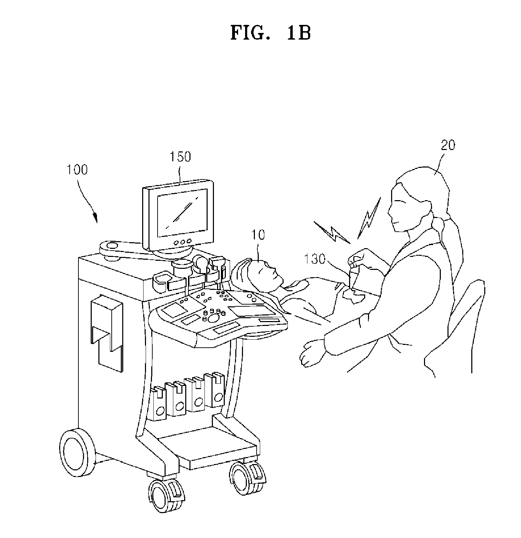

FIGS. 1A and 1B are images of the ultrasound apparatus 100 including a plurality of probes, according to an embodiment of the present invention.

Reffering to FIG. 1A, the ultrasound apparatus 100 may include a wired probe 110 and a wireless probe 130, according to an exemplary embodiment.

As illustrated in FIG. 1A, the ultrasound apparatus 100 according to the current exemplary embodiment may include at least one wired probe 110, at least one wireless probe 130, and a display 150.

A user 20 may obtain an ultrasound image of an object 10 by using the wired or wireless probe 110 or 130. The obtained ultrasound image may be output via the display 150. In other words, the ultrasound apparatus 100 according to the current exemplary embodiment may generate the ultrasound image of the object 10 by using both of the wired and wireless probes 110 and 130.

However, the ultrasound apparatus 100 according to the present embodiment is not limited to FIG. 1A, and may include the greater or less number of wired probes or wireless probes than those of the ultrasound apparatus 100 shown in FIG. 100.

As illustrated in FIG. 1A, the ultrasound apparatus 100 according to the present embodiment may generate a plurality of ultrasound images by using the plurality of probes, and thus may simultaneously obtain the ultrasound images with different angles with respect to an object. Also, the ultrasound apparatus 100 according to the present embodiment may simultaneously monitor and manage ultrasound images that are scanned by a plurality of users.

As illustrated in FIG. 1B, the ultrasound apparatus 100 according to the present embodiment may include the wireless probe 130 and the display 150.

The user 20 may obtain an ultrasound image of the object 10 by using the wireless probe 130. The obtained ultrasound image may be output via the display 150. Referring to FIG. 1B, the ultrasound apparatus 100 includes only one wireless probe 130, but the ultrasound apparatus 100 according to the present embodiment may include a plurality of wireless probes. The ultrasound apparatus 100 may generate a plurality of ultrasound images of the object 10 by simultaneously using the plurality of wireless probes.

In the present embodiment, the wireless probe 130 may be simultaneously connected to the ultrasound apparatus 100 and at least one external device. The wireless probe may receive an ultrasound response signal that is reflected from the object 10 in response to an ultrasound signal that was transmitted to the object 10. The wireless probe 130 may transmit the ultrasound response signal to the at least one external device.

As illustrated in FIG. 1B, the ultrasound apparatus 100 according to the present embodiment may obtain ultrasound data by using a probe and may simultaneously transmit the ultrasound data to the at least one external device, so that the ultrasound apparatus 100 and the at least one external device may provide an equal image to the user 20 or may provide different images that are differently processed, to the user 20.

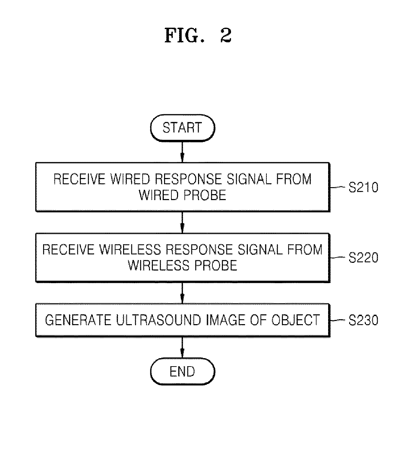

FIG. 2 is a flowchart of a method of generating an ultrasound image, according to an exemplary embodiment. Referring to FIG. 2, the method according to the current exemplary embodiment includes operations performed in time series by the ultrasound apparatus 100 illustrated in FIG. 1A.

In operation S210, the ultrasound apparatus 100 receives, from at least one wired probe 110, a wired response signal corresponding to a first ultrasound response signal reflected from the object 10. The at least one wired probe 110 may transmit an ultrasound signal to the object 10, and may receive the first ultrasound response signal reflected from the object 10. The wired response signal may be a signal which is the same as the first ultrasound response signal, or a signal modulated from the first ultrasound response signal in order to be transmitted to the ultrasound apparatus 100.

In operation S220, the ultrasound apparatus 100 receives, from at least one wireless probe 130, a wireless response signal corresponding to a second ultrasound response signal reflected from the object 10. The at least one wireless probe 130 may transmit an ultrasound signal to the object 10, and may receive the second ultrasound response signal reflected from the object 10. The wireless response signal may be a signal which is the same as the second ultrasound response signal, or a signal modulated from the second ultrasound response signal in order to be transmitted to the ultrasound apparatus 100.

The ultrasound apparatus 100 may simultaneously receive the wired and wireless response signals from the at least one wired probe 110 and the at least one wireless probe 130. A fact that the ultrasound apparatus 100 simultaneously receives the wired and wireless response signals means that the wired and wireless response signals are received simultaneously at a certain point of time. In other words, the user 20 of the ultrasound apparatus 100 according to the current exemplary embodiment may simultaneously use the wired and wireless probes 110 and 130.

In operation S230, the ultrasound apparatus 100 generates an ultrasound image of the object 10 by using the wired and wireless response signals. The ultrasound apparatus 100 may generate a wired ultrasound image of the object 10 by using the wired response signal, and may generate a wireless ultrasound image of the object 10 by using the wireless response signal.

In order to allow the ultrasound apparatus 100 to generate the ultrasound image by using the wired and wireless response signals, wired ultrasound data and wireless ultrasound data should be generated by performing signal processing such as reception beam forming on the wired and wireless response signals.

If the wired and wireless response signals are simultaneously received, the ultrasound apparatus 100 according to the current exemplary embodiment may perform signal processing on the wired response signal and signal processing on the wireless response signal in parallel by using two signal processors, e.g., a first signal processor and a second signal processor. Alternatively, the ultrasound apparatus 100 may perform signal processing on the wired and wireless response signals in a time-sharing manner by using one signal processor. For example, one signal processor may alternately perform signal processing on the wired response signal and signal processing on the wireless response signal in a predetermined time cycle.

The wired and wireless ultrasound images may be different ultrasound images. For example, the wired and wireless ultrasound images may be different types of ultrasound images selected from the group consisting of an amplitude (A) mode image, a brightness (B) mode image, a motion (M) mode image, a Doppler spectrum image, a color Doppler image, and an elasticity image.

Alternatively, the wired and wireless ultrasound images may be ultrasound images of different cross-sections of the object 10. For example, the wired ultrasound image may be an ultrasound image of a sagittal plane of the object 10, and the wireless ultrasound image may be an ultrasound image of a coronal plane of the object 10.

The ultrasound apparatus 100 may respectively output the generated wired and wireless ultrasound images via first and second displays, or may respectively display the wired and wireless ultrasound images on first and second areas included one display.

Also, the ultrasound apparatus 100 according to the current exemplary embodiment may generate a composite ultrasound image of the object 10 by combining the different wired and wireless ultrasound images.

For example, if one wired ultrasound image and two wireless ultrasound images are obtained as ultrasound images of different cross-sections of the object 10, a 3D ultrasound image of the object 10 may be generated by combining these three ultrasound images.

As another example, if the wired ultrasound image is a B-mode image and the wireless ultrasound image is an elasticity image, a composite ultrasound image clearly showing anatomical and mechanical properties inside the object 10 may be generated by combining the B-mode image and the elasticity image.

In addition, a composite ultrasound image clearly showing properties of the object 10 may be generated by combining different types of ultrasound images within a range well-known to one of ordinary skill in the art.

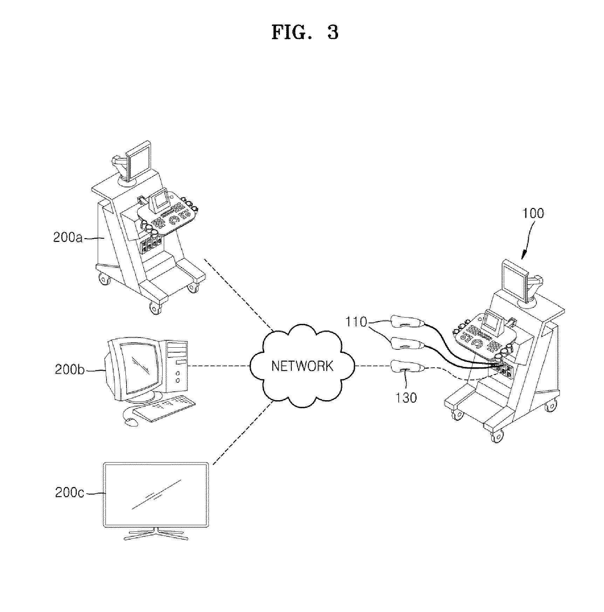

FIG. 3 is an image of the ultrasound apparatus 100 including the wireless probe 130 wirelessly connected to external devices 200a, 200b, and 200c, according to another exemplary embodiment.

The ultrasound apparatus 100 according to the current exemplary embodiment may include at least one wireless probe 130 connected to the external devices 200a, 200b, and 200c via a network. Although only one wireless probe 130 is illustrated in FIG. 3, a plurality of wireless probes 130 may be individually connected to the external devices 200a, 200b, and 200c via a network. Also, although the ultrasound apparatus 100 includes both of the wired probes 110 and the wireless probe 130 in FIG. 3, it would be understood by one of ordinary skill in the art that the ultrasound apparatus 100 may include only the wireless probe 130.

The external devices 200a, 200b, and 200c may include an external server, a magnetic resonance imaging (MRI) apparatus, a computerized tomography (CT) apparatus, an X-ray apparatus, and a mobile apparatus as well as an ultrasound apparatus (200a), a computer (200b), and a digital television (TV) (200c) illustrated in FIG. 3. However, the external devices are not limited thereto.

The wireless probe 130 may transmit a wireless response signal of the object 10 to the ultrasound apparatus 100 and the external devices 200a, 200b, and 200c. The ultrasound apparatus 100 and the external devices 200a, 200b, and 200c may individually generate wireless ultrasound images by processing the received wireless response signal. The wireless ultrasound images individually generated by the ultrasound apparatus 100 and the external devices 200a, 200b, and 200c may be different types of ultrasound images. For example, the ultrasound apparatus 100 may generate a B-mode image, and the computer 200b may generate a color Doppler image. In other words, the wireless response signal obtained by one wireless probe 130 may be transmitted to a plurality of external devices 200a, 200b, and 200c as well as the ultrasound apparatus 100, and the user 20 of the ultrasound apparatus 100 and users of the plurality of external devices 200a, 200b, and 200c may individually generate their desired ultrasound images by using the wireless response signal. In other words, the wireless probe 130 of the ultrasound apparatus 100 according to the current exemplary embodiment may be shared with the plurality of external devices 200a, 200b, and 200c.

The wireless probe 130 may simultaneously transmit the wireless response signal to the ultrasound apparatus 100 and the external devices 200a, 200b, and 200c. A fact that the wireless probe 130 simultaneously transmits the wireless response signal to the ultrasound apparatus 100 and the external devices 200a, 200b, and 200c means that the wireless response signal is simultaneously transmitted to the ultrasound apparatus 100 and the external devices 200a, 200b, and 200c at a certain point of time.

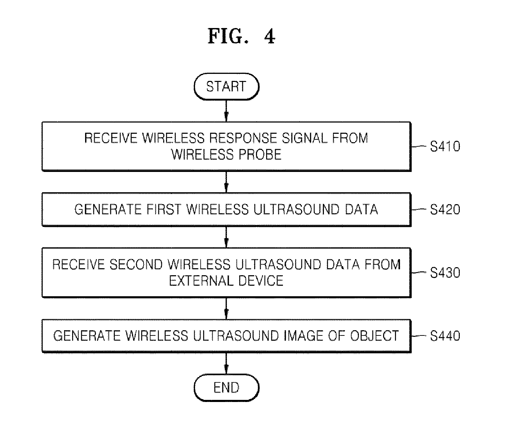

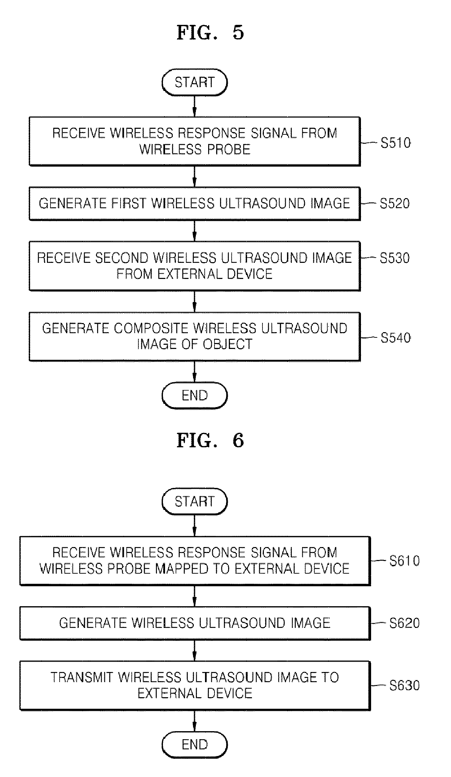

For example, the wireless probe 130 may simultaneously transmit the wireless response signal to the ultrasound apparatus 100 and the external devices 200a, 200b, and 200c by broadcasting the wireless response signal via a network, or may simultaneously transmit the wireless response signal via wireless communication channels individually generated between the ultrasound apparatus 100, the external devices 200a, 200b, and 200c, and the wireless probe 130. Since the wireless probe 130 is shared with the plurality of external devices 200a, 200b, and 200c, a time taken to generate an ultrasound image, and the complexity of calculation to generate an ultrasound image may be reduced. A description thereof is now provided with reference to FIGS. 4 and 5.

FIG. 4 is a flowchart of a method of generating an ultrasound image, according to another exemplary embodiment.

In operation S410, the ultrasound apparatus 100 receives a wireless response signal from at least one wireless probe 130. The at least one wireless probe 130 may transmit an ultrasound signal to the object 10, may receive an ultrasound response signal reflected from the object 10, and may obtain the wireless response signal corresponding to the ultrasound response signal.

In operation S420, the ultrasound apparatus 100 generates first wireless ultrasound data based on a first part of the wireless response signal. The wireless response signal is generated by using wireless response signals received by the wireless probe 130 via a plurality of transducers, and the ultrasound apparatus 100 may generate the first wireless ultrasound data based on the wireless response signals corresponding to some of the plurality of transducers.

In operation S430, the ultrasound apparatus 100 receives second wireless ultrasound data generated based on a second part of the wireless response signal, from an external device. In more detail, the at least one wireless probe 130 of the ultrasound apparatus 100 transmits the wireless response signal of the object 10 to the external device. The external device may receive the wireless response signal, may generate the second wireless ultrasound data based on the second part of the wireless response signal, and then may transmit the generated second wireless ultrasound data to the ultrasound apparatus 100.

In operation S440, the ultrasound apparatus 100 generates a wireless ultrasound image of the object 10 by using the first and second wireless ultrasound data. Since a wireless ultrasound data corresponding to the whole wireless response signal is generated by combining the first and second wireless ultrasound data, the ultrasound apparatus 100 may generate the wireless ultrasound image based on the first and second wireless ultrasound data.

Since the ultrasound apparatus 100 according to the current exemplary embodiment generates a wireless ultrasound image by processing only a part of a wireless response signal so as to generate wireless ultrasound data, and receiving wireless ultrasound data of another part of the wireless response signal from an external device, without processing the whole wireless response signal, a time taken to generate the wireless ultrasound image may be reduced by at least 1/2. If the number of external devices is large, the time taken to generate the wireless ultrasound image may be further reduced.

FIG. 5 is a flowchart of a method of generating an ultrasound image, according to another exemplary embodiment.

In operation S510, the ultrasound apparatus 100 receives a wireless response signal from at least one wireless probe 130.

In operation S520, the ultrasound apparatus 100 generates a first wireless ultrasound image based on the wireless response signal. The ultrasound apparatus 100 may generate wireless ultrasound data by processing the wireless response signal, and may generate the first wireless ultrasound image based on the wireless ultrasound data.

In operation S530, the ultrasound apparatus 100 receives a second wireless ultrasound image generated based on the wireless response signal, from an external device. In more detail, the at least one wireless probe 130 of the ultrasound apparatus 100 transmits the wireless response signal of the object 10 to the external device. The external device may receive the wireless response signal, may generate the second wireless ultrasound image based on the wireless response signal, and then may transmit the generated second wireless ultrasound image to the ultrasound apparatus 100.

In operation S540, the ultrasound apparatus 100 generates a composite wireless ultrasound image of the object 10 by combining the first and second wireless ultrasound images. For example, if the first wireless ultrasound image is a B-mode image and the second wireless ultrasound image is an elasticity image, the ultrasound apparatus 100 may generate a composite wireless ultrasound image clearly showing anatomical and mechanical properties inside the object 10 by combining the B-mode image and the elasticity image.

Since the ultrasound apparatus 100 according to the current exemplary embodiment generates a composite wireless ultrasound image by generating only a first wireless ultrasound image, and receiving a second wireless ultrasound image from an external device, without generating both of the first and second wireless ultrasound images, a time taken to generate the composite wireless ultrasound image may be reduced by at least 1/2. If the number of external devices is large, the time taken to generate the composite wireless ultrasound image may be further reduced.

Referring back to FIG. 3, the ultrasound apparatus 100 and the external devices 200a, 200b, and 200c for sharing the wireless probe 130 may have different control authorities on the wireless probe 130. The ultrasound apparatus 100 may have a master control authority on the wireless probe 130, and the external devices 200a, 200b, and 200c may have a slave control authority on the wireless probe 130. The master control authority may be set to control all functions of the wireless probe 130, for example, an irradiation timing of an ultrasound signal, a focal depth of the ultrasound signal, and an irradiation direction of the ultrasound signal. On the other hand, the slave control authority may be set to control only a part of the functions of the wireless probe 130, for example, only the irradiation timing of the ultrasound signal. The slave control authority may be variously set by the user 20 of the ultrasound apparatus 100.

The ultrasound apparatus 100 having the master control authority may transfer the master control authority to at least one of the external devices 200a, 200b, and 200c. The external device that has received the master control authority may have the master control authority on the wireless probe 130, and the ultrasound apparatus 100 that has transferred the master control authority may have the slave control authority. Alternatively, according to a setup of the user 20, both of the external device that has received the master control authority, and the ultrasound apparatus 100 that has transferred the master control authority, may have the master control authority.

FIG. 6 is a flowchart of a method of generating an ultrasound image, according to another exemplary embodiment. Although the descriptions of FIGS. 4 and 5 are provided on the assumption that an external device is able to generate ultrasound data or an ultrasound image by processing an ultrasound response signal, in FIG. 6, the external device is not able to generate ultrasound data or an ultrasound image by processing an ultrasound response signal. In other words, in FIG. 6, the external device does not include an ultrasound apparatus that is able to processing an ultrasound response signal.

In operation S610, the ultrasound apparatus 100 receives a wireless response signal of the object 10 from the wireless probe 130 mapped to an external device. The wireless probe 130 may transmit a message indicating the type of an ultrasound image desired by the external device, to the ultrasound apparatus 100 while transmitting the wireless response signal to the ultrasound apparatus 100.

If the external device is a computer, a user of the computer may obtain the wireless response signal of the object 10 by using the wireless probe 130 mapped to the computer. Then, the wireless probe 130 may transmit the wireless response signal to the ultrasound apparatus 100. If the wireless probe 130 is able to generate wireless ultrasound data by using the wireless response signal, the wireless probe 130 may transmit the wireless ultrasound data to the ultrasound apparatus 100.

In operation S620, the ultrasound apparatus 100 generates a wireless ultrasound image of the object 10 by using the wireless response signal. The wireless ultrasound image may be the type of an ultrasound image corresponding to the message received from the wireless probe 130.

In operation S630, the ultrasound apparatus 100 transmits the wireless ultrasound image to the external device. As such, the user of the computer may view an ultrasound image of the object 10.

In other words, the ultrasound apparatus 100 according to the current exemplary embodiment may function as a server for transmitting an ultrasound image to external devices, instead of an ultrasound apparatus. If a hospital has one ultrasound apparatus 100 functioning as a server and a plurality of wireless probes 130 mapped to a plurality of external devices, ultrasound images of a large number of patients may be generated within a short time, and thus costs may be greatly reduced.

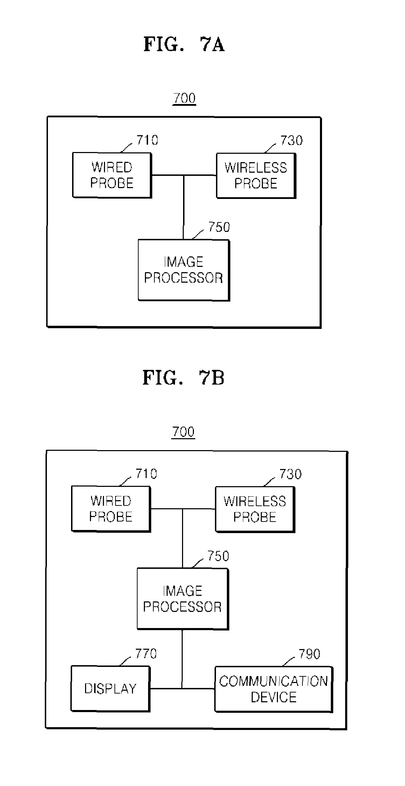

FIG. 7A is a block diagram of an ultrasound apparatus 700 according to an exemplary embodiment.

Referring to FIG. 7A, the ultrasound apparatus 700 according to the current exemplary embodiment may include a wired probe 710, a wireless probe 730, and an image processor 750.

In the ultrasound apparatus 700, one or more wired probes 710 and one or more wireless probes 730 may exist, and the image processor 750 may be formed as a micro processor.

The wired probe 710 obtains a wired response signal corresponding to a first ultrasound response signal reflected from an object.

The wireless probe 730 obtains a wireless response signal corresponding to a second ultrasound response signal reflected from the object. The wireless probe 730 may be connected to external devices via a network and may transmit the wireless response signal to the external devices. Alternatively, the wireless probe 730 may be mapped to the external device.

The image processor 750 generates an ultrasound image of the object 10 by using the wired and wireless response signals received from the wired and wireless probes 710 and 730. The image processor 750 may generate a composite ultrasound image by combining a wired ultrasound image generated based on the wired response signal, and a wireless ultrasound image generated based on the wireless response signal.

The image processor 750 may include a signal processor (not shown) for generating wired and wireless ultrasound data by processing the wired and wireless response signals, and an image generator (not shown) for generating the wired and wireless ultrasound images based on the wired and wireless ultrasound data.

The image processor 750 may include one signal processor or two signal processors. The one signal processor may generate the wired and wireless ultrasound data corresponding to the wired and wireless response signals by processing the wired and wireless response signals in a time-sharing manner. Also, the two signal processors, e.g., a first signal processor and a second signal processor, may individually generate the wired and wireless ultrasound data by processing the wired and wireless response signals.

The image processor 750 may generate first wireless ultrasound data based on a first part of the wireless response signal received from the wireless probe 730, may receive second wireless ultrasound data corresponding to a second part of the wireless response signal from the external device, and then may generate the wireless ultrasound image of the object based on the first and second wireless ultrasound data.

Also, the image processor 750 may generate a first wireless ultrasound image based on the wireless response signal received from the wireless probe 730, may receive a second wireless ultrasound image corresponding to the wireless response signal from the external device, and then may generate a composite wireless ultrasound image of the object by combining the first and second wireless ultrasound images.

FIG. 7B is a block diagram of the ultrasound apparatus 700 according to another exemplary embodiment.

Referring to FIG. 7B, the ultrasound apparatus 700 according to the current exemplary embodiment may include the wired probe 710, the wireless probe 730, the image processor 750, a display 770, and a communication device 790. The wired probe 710, the wireless probe 730, and the image processor 750 are described above in relation to FIG. 7A, and thus detailed descriptions thereof are not provided here.

The display 770 outputs the ultrasound image generated by the image processor 750. The display 770 may display the wired and wireless ultrasound images generated by the image processor 750, on first and second areas of the display 770. Alternatively, the display 770 may include first and second displays for respectively outputting the wired and wireless ultrasound images generated by the image processor 750. The display 770 may include a cathode-ray tube (CRT), a liquid crystal display (LCD), a plasma display panel (PDP), an organic light emitting diode (OLED), a field emission display (FED), a light emitting diode (LED), a vacuum fluorescent display (VFD), a digital light processing (DLP) display, a primary flight display (PFD), a 3D display, a transparent display, etc., or may include other various displays within a range well-known to one of ordinary skill in the art.

The communication device 790 may transmit the ultrasound image generated by the image processor 750 to the external device.

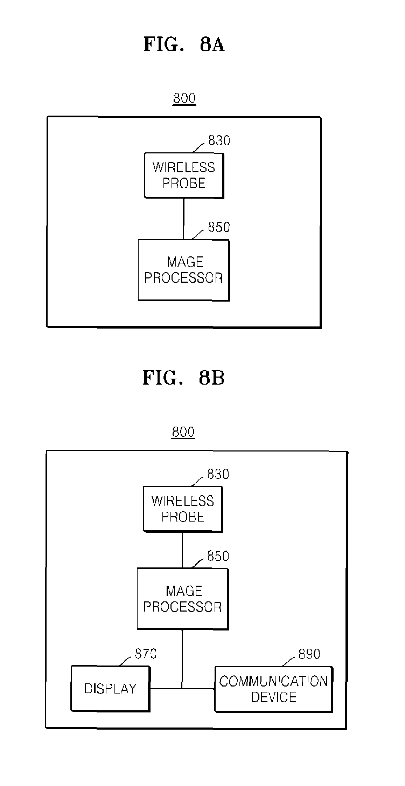

FIG. 8A is a block diagram of an ultrasound apparatus 800 according to another embodiment of the present invention.

Referring to FIG. 8A, the ultrasound apparatus 800 according to the present embodiment may include a wireless probe 830 and an image processor 850.

In the ultrasound apparatus 800, one or more wireless probes 830 may exist, and the image processor 850 may be formed as a micro processor.

The wireless probe 830 obtains a wireless response signal corresponding to a second ultrasound response signal reflected from an object.

The wireless probe 730 obtains a wireless response signal corresponding to a second ultrasound response signal reflected from the object. The wireless probe 830 may be connected to external devices via a network and may transmit the wireless response signal to the external devices. Alternatively, the wireless probe 830 may be mapped to the external device.

The image processor 850 generates an ultrasound image of the object by using the wireless response signal received from the wireless probe 830. The image processor 850 may generate a composite ultrasound image by combining a plurality of wireless ultrasound images generated based on wireless response signals received from a plurality of wireless probes.

The image processor 850 may include a signal processor (not shown) for generating wireless ultrasound data by processing the wireless response signal, and an image generation unit (not shown) for generating the wireless ultrasound image based on the wireless ultrasound data.

The image processor 850 may generate first wireless ultrasound data based on a first part of the wireless response signal received from the wireless probe 830, may receive second wireless ultrasound data corresponding to a second part of the wireless response signal from the external device, and then may generate the wireless ultrasound image of the object based on the first and second wireless ultrasound data.

Also, the image processor 850 may generate a first wireless ultrasound image based on the wireless response signal received from the wireless probe 830, may receive a second wireless ultrasound image corresponding to the wireless response signal from the external device, and then may generate a composite wireless ultrasound image of the object by combining the first and second wireless ultrasound images.

FIG. 8B is a block diagram of the ultrasound apparatus 800 according to another embodiment of the present invention.

Referring to FIG. 8B, the ultrasound apparatus 800 according to the present embodiment may include the wireless probe 830 and the image processor 850, and may further include a display 870, and a communication device 890. The wireless probe 830 and the image processor 850 are described above in relation to FIG. 7A, and thus detailed descriptions thereof are not provided here.

The display 870 outputs the ultrasound image generated by the image processor 850. The display 870 may display a plurality of wireless ultrasound images generated by the image processor 850, on first and second areas of the display 870. Alternatively, the display 870 may include a plurality of displays for respectively outputting the wireless ultrasound images generated by the image processor 850. The display 870 may include a CRT, an LCD, a PDP, an OLED, an FED, an LED, a VFD, a DLP display, a PFD, a 3D display, a transparent display, etc., or may include other various displays within a range well-known to one of ordinary skill in the art.

The communication device 890 may transmit the ultrasound image generated by the image processor 850 to the external device.

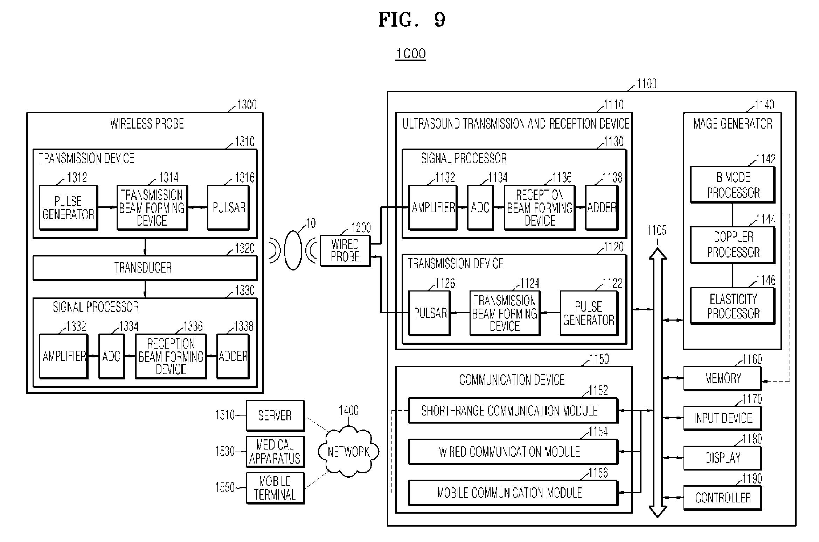

FIG. 9 is a block diagram of an ultrasound apparatus 1000 according to another exemplary embodiment.

The ultrasound apparatus 1000 illustrated in FIG. 8 may include a main body 1100, a wired probe 1200, and a wireless probe 1300. The main body 1100 may include an ultrasound transmission and reception device 1110, an image generator 1140, a communication device 1150, a memory 1160, an input device 1170, a display 1180, and a controller 1190, which may be connected to each other via a bus 1105.

The ultrasound apparatus 1000 may be a portable type as well as a cart type. Examples of the portable ultrasound apparatus include a picture archiving and communication system (PACS) viewer, a smart phone, a laptop computer, a personal digital assistant (PDA), and a tablet personal computer (PC), but are not limited thereto.

The wired probe 1200 transmits an ultrasound signal to the object 10 according to a driving signal provided from the ultrasound transmission and reception device 1110, and receives an ultrasound response signal reflected from the object 10. The wired probe 1200 includes a plurality of transducers that vibrate according to a transmitted electrical signal and generate ultrasound waves corresponding to acoustic energy. The ultrasound apparatus 1000 may include a plurality of wired probes 1200 according to a design.

A transmission device 1120 provides a driving signal to the wired probe 1200, and includes a pulse generator 1122, a transmission beam forming device 1124, and a pulsar 1126. The pulse generator 1122 generates pulses for generating transmission ultrasound waves according to a predetermined pulse repetition frequency (PRF), and the transmission beam forming device 1124 applies to the pulses a delay time for determining transmission directionality. The pulses to which the delay time is applied individually correspond to a plurality of piezoelectric vibrators included in the wired probe 1200. The pulsar 1126 provides a driving signal (or a driving pulse) to the wired probe 1200 at a timing corresponding to each pulse to which the delay time is applied.

A signal processor 1130 generates ultrasound data by processing a response signal received from the wired probe 1200, and may include an amplifier 1132, an analog-digital converter (ADC) 1134, a reception beam forming device 1136, and an adder 1138. The amplifier 1132 amplifies each channel of the response signal, and the ADC 1134 analog-digital converts the amplified response signal. The reception beam forming device 1136 applies to the converted response signal a delay time for determining reception directionality, and the adder 1138 generates ultrasound data by summing the response signals processed by the reception beam forming device 1136.

The image generator 1140 generates an ultrasound image by performing scan conversion on the ultrasound data generated by the ultrasound transmission and reception device 1110. The ultrasound image may include a gray-scale ultrasound image obtained by scanning an object in an amplitude (A) mode, a brightness (B) mode, and a motion (M) mode, and a Doppler image representing motion of the object. The Doppler image may include a blood flow Doppler image representing the flow of blood (also referred to as a color Doppler image), a tissue Doppler image representing motion of tissues, and a spectral Doppler image representing a velocity of an object in a waveform. The signal processor 1130 and the image generator 1140 may be included in the image processor 750 illustrated in FIGS. 7A and 7B.

A B mode processor 1142 may extract a B mode component from the ultrasound data, and may generate an ultrasound image representing the intensity of a signal as a brightness based on the B mode component.

A Doppler processor 1144 may extract a Doppler component from the ultrasound data, and may generate a Doppler image representing the motion of the object as a color or a waveform based on the extracted Doppler component.

An elasticity processor 1146 may extract a strain of the object from the ultrasound data, and may generate an elasticity image representing mechanical characteristics of the object based on the extracted strain.

The image generator 1140 may generate a 3D ultrasound image by rendering volume data, and may generate a composite ultrasound image by combining different ultrasound images. Furthermore, the image generator 1140 may represent various types of additional information as text, graphic, etc. on the ultrasound image.

The memory 1160 stores various types of information processed by the ultrasound apparatus 1000. For example, the memory 1160 may store ultrasound data to be input and output, medical data related to diagnosis of the object, e.g., an ultrasound image, and an algorithm or program executed in the ultrasound apparatus 1000.

The memory 1160 may include various storing media such as a flash memory, a hard disk, and an electrically erasable programmable read-only memory (EEPROM). Also, the ultrasound apparatus 1000 may operate a web storage or a cloud server for performing the storing function of the memory 1160 on the web.

The input device 1170 may refer to a device for receiving an input from a user for controlling the ultrasound apparatus 1000. The input device 1170 may include hardware components such as a key pad, a mouse, a touch panel, a touch screen, a trackball, and a jog switch. The input device 1170 is not limited thereto, and may further include various input means such as an electrocardiogram (ECG) module, a respiratory measurement module, a speech recognition sensor, a gesture recognition sensor, a fingerprint sensor, an iris recognition sensor, a depth sensor, and a distance sensor.

The display 1180 displays and outputs the generated ultrasound image. In addition to the ultrasound image, the display 1180 may display various types of information processed by the ultrasound apparatus 1000, on a screen via a graphic user interface (GUI). The ultrasound apparatus 1000 may include two or more displays 1180 according to a design.

The controller 1190 controls entire operations of the ultrasound apparatus 1000. In other words, the controller 1190 may control operations among the wired probe 1200, the ultrasound transmission and reception device 1110, the image generator 1140, the communication device 1150, the memory 1160, the input device 1170, and the display 1180.

The communication device 1150 may exchange data with a hospital server or another medical apparatus connected via a PACS in a hospital, and may perform data communications according to the Digital Imaging and Communications in Medicine (DICOM) standard.

As illustrated in FIG. 9, the communication device 1150 is connected to a network 1400 by wired means or wirelessly, and thus may communicate with external devices such as a server 1510, a medical apparatus 1530, and a mobile terminal 1550.

In more detail, the communication device 1150 may transmit and receive data related to diagnosis of the object 10 via the network 1400, and may transmit and receive a medical image captured by the medical apparatus 1530, e.g., a CT, MRI, or X-ray image. Furthermore, the communication device 1150 may receive a diagnosis history or a treatment schedule of a patient from the server 1550 and may utilize them to diagnose the object 10. Also, the communication device 1150 may perform data communications with the server 1510 or the medical apparatus 1530 inside a hospital, and the mobile terminal 1550 of a doctor or a customer, for example, a mobile phone, a PDA, or a laptop computer. The communication device 1150 may include one or more components for allowing communications with an external device, for example, a short-range communication module 1152, a wired communication module 1154, and a mobile communication module 1156.

The short-range communication module 1152 refers to a module for communications within a predetermined distance. Short-range communication technologies according to an exemplary embodiment include wireless local area network (LAN), wireless fidelity (Wi-Fi), Bluetooth, Zigbee, Wi-Fi direct (WFD), ultra wideband (UWB), IrDA (infrared data association), Bluetooth low energy (BLE), and near field communication (NFC), and are not limited thereto.

The wired communication module 1154 refers to a module for communications using electrical signals or optical signals. Wired communication technologies according to an exemplary embodiment include communications using a pair cable, a coaxial cable, a fiber optic cable, and an Ethernet cable.

The mobile communication module 1156 transmits and receives wireless signals to and from at least one of a base station, an external terminal, and a server in a mobile communication network. Here, the wireless signals may include various types of data according to transmission and reception of a voice call signal, a video call signal, or a text/multimedia message.

Some or all of the wired probe 1200, the ultrasound transmission and reception device 1110, the image generator 1140, the communication device 1150, the memory 1160, the input device 1170, the display 1180, and the controller 1190 may be driven by a software module, but are not limited thereto. Some of the above-mentioned components may be driven by a hardware module. Also, at least one of the ultrasound transmission and reception device 1110, the image generator 1140, and the communication device 1150 may be included in the controller 1190, and is not limited thereto.

The wireless probe 1300 includes a plurality of transducers, and may include a part or the whole ultrasound transmission and reception device 1110 according to a design.

The wireless probe 1300 may include a transmission device 1310, a transducer 1320, and a signal processor 1330. A description of the transmission device 1310, the transducer 1320 and the signal processor 1330 will be omitted here, since the description with respect to the identical components is made above.

The wireless probe 1300 transmits an ultrasound signal to and receives a response signal from the object 10, and may wirelessly transmit the response signal or ultrasound data to the main body 1100. Also, the wireless probe 1300 may be connected to external devices including the server 1510, the medical apparatus 1530, and the mobile terminal 1550 via the network 1400, and may wirelessly transmit the response signal to the external devices.

The exemplary embodiments can be written as computer programs and can be implemented in general-use digital computers that execute the programs using a computer readable recording medium.

Examples of the computer readable recording medium include magnetic storage media (e.g., ROM, floppy disks, hard disks, etc.), optical recording media (e.g., CD-ROMs, or DVDs), etc.

It should be understood that the exemplary embodiments described therein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the spirit and scope of the exemplary embodiments as defined by the following claims.

## Claims

1. A method of generating an ultrasound image in an ultrasound apparatus connected with at least one wired probe and at least one wireless probe, the method comprising:
receiving, from the at least one wired probe, a wired response signal corresponding to a first ultrasound response signal reflected from an object;
receiving, from the at least one wireless probe, a wireless response signal corresponding to a second ultrasound response signal reflected from the object; and
generating an ultrasound image of the object based on the wired response signal and the wireless response signals.

2. The method of claim 1, wherein the generating of the ultrasound image comprises generating a wired ultrasound image of the object based on the wired response signal, and generating a wireless ultrasound image of the object based on the wireless response signal.

3. The method of claim 2, further comprising outputting the wired ultrasound image on a first display and outputting the wireless ultrasound image on a second display.

4. The method of claim 2 or 3, wherein the wireless ultrasound image comprises an ultrasound image which is different from the wired ultrasound image.

5. The method of claim 4, wherein the generating of the ultrasound image comprises generating a composite ultrasound image of the object by combining the wired ultrasound image and the wireless ultrasound image.

6. The method of claim 2, 3, 4 or 5, wherein the generating of the ultrasound image comprises:
generating wired ultrasound data corresponding to the wired response signal and wireless ultrasound data corresponding to the wireless response signal by processing the wired response signal and the wireless response signals in a time-sharing manner; and
generating the wired ultrasound image based on the wired ultrasound data and generating the wireless ultrasound image based on the wireless ultrasound data.

7. The method of any of claims 2-6, wherein the generating of the ultrasound image comprises:
generating wired ultrasound data from the wired response signal based on a first signal processor and generating wireless ultrasound data from the wireless response signal based on a second signal processor; and
generating the wired ultrasound image based on the wired ultrasound data and generating the wireless ultrasound image based on the wireless ultrasound data.

8. The method of any of claims 1-7, wherein the at least one wireless probe transmits the wireless response signal to an external device.

9. The method of claim 8, wherein the generating of the ultrasound image comprises:
generating first wireless ultrasound data based on a first part of the wireless response signal received from the at least one wireless probe;
receiving from the external device second wireless ultrasound data generated based on a second part of the wireless response signal transmitted to the external device; and
generating a wireless ultrasound image of the object by combining the first wireless ultrasound data and the second wireless ultrasound data.

10. The method of claim 8 or 9, wherein the generating of the ultrasound image comprises:
generating a first wireless ultrasound image based on the wireless response signal received from the at least one wireless probe;
receiving from the external device a second wireless ultrasound image generated based on the wireless response signal transmitted to the external device; and
generating a composite wireless ultrasound image of the object by combining the first wireless ultrasound image and the second wireless ultrasound image.

11. The method of claim 8, 9 or 10 wherein the ultrasound apparatus has a master control authority on the at least one wireless probe, and
wherein the external device has a slave control authority on the at least one wireless probe.

12. The method of any of claims 2-11, wherein the at least one wireless probe is mapped to an external device, and
wherein the method further comprises transmitting the wireless ultrasound image to the external device.

13. A non-transitory computer-readable recording medium having recorded thereon a computer program for executing the method of any one of claims 1 to 12.

14. An ultrasound apparatus comprising:
at least one wired probe configured to obtain a wired response signal corresponding to a first ultrasound response signal reflected from an object;
at least one wireless probe configured to obtain a wireless response signal corresponding to a second ultrasound response signal reflected from the object; and
an image processor configured to generate an ultrasound image of the object based on the wired response signal and the wireless response signal received from the at least one wired probe and the at least one wireless probe.

15. The ultrasound apparatus of claim 14, wherein the image processor is configured to generate a wired ultrasound image of the object based on the wired response signal, and generate a wireless ultrasound image of the object based on the wireless response signal.
